# EUROPEAN PATENT SPECIFICATION

(11) **EP 2 498 752 B1**
(45) Date of publication and mention of the grant of the patent: **12.04.2017**
(21) Application number: 10796170.8
(22) Date of filing: 11.11.2010
(51) Int. Cl.: A61K 8/36, A61K 8/365, A61K 31/19, A61K 47/12, A61K 47/14, A61K 9/00, A61Q 3/00, A61Q 17/00, A61M 35/00

(54) **COMPOSITION FOR TOPICAL APPLICATION, USES THEREOF, APPLICATOR DEVICE AND KIT OF PARTS**
ZUSAMMENSETZUNG ZUR TOPISCHEN ANWENDUNG, IHRE VERWENDUNG, APPLIKATOR UND TEILESATZ
COMPOSITION POUR APPLICATION TOPIQUE, SES UTILISATIONS, DISPOSITIF D'APPLICATION ET NÉCESSAIRE ASSOCIÉ

(30) Priority: 11.11.2009 NL 2003786
(43) Date of publication of application: 19.09.2012
(73) Proprietor: YouMedical B.V., 1059 AT Amsterdam (NL)
(72) Inventor: STAL, Robert, Sebastian, NL-1018 HA Amsterdam (NL)
(74) Representative: V.O.
(86) International application number: PCT/NL2010/050750
(87) International publication number: WO 2011/059324

(56) References cited:
- WO-A1-92/13529
- WO-A2-00/15202
- WO-A2-2004/021968
- WO-A2-2008/128627
- CA-C- 1 330 198
- US-A1- 2002 183 387
- US-A1- 2007 104 772
- US-A1- 2008 112 908
- US-A1- 2008 311 231

## Description

### FIELD OF THE INVENTION

The invention relates to a composition for topical application. The invention further relates to various uses for the treatment of fungal infections, including and especially focussing on microbiological infections of the nail (onychomycosis). The invention further relates to an applicator device and kit of parts comprising such a composition.

### BACKGROUND OF THE INVENTION

Onychomycosis is a wide-spread microbiological infection of the keratin of the nail. The fungi are able to enter the nail through microscopic cracks in the nail. Once in the nail the fungus uses the macroscopic molecules in the nail to feed itself and alter the environment to its own advantage. One of these alterations includes raising the pH of the nail to an alkaline level. This creates a favourable environment for the fungus in which it can multiply sexually instead of asexually.

US 2008/0112908 discloses anti-fungal nail lacquer comprising glacial acetic acrid.

### OBJECT AND SUMMARY OF THE INVENTION

It is an object of the invention to provide a safe and effective treatment for onychomycosis.

The invention relates to a composition for topical application, comprising at least one physiologically acceptable carboxylic acid capable of reducing the pH in keratinous tissue, in particular nails, of a treated person below 4.5, preferably in the range of 1.5 - 4.5, wherein said at least one carboxylic acid is selected from the group consisting of lactic acid, malic acid, tartaric acid, citric acid, acetic acid, propionic acid, isopropionic acid, oxalic acid, glutaric acid, adipic acid, and glycolic acid; and a physiologically acceptable carrier comprising at least one C1-C4 alkyl ester of lactic acid; and wherein the carboxylic acid is present in a quantity of at least 1 % by weight, and wherein the C1-C4 alkyl ester is present in a quantity of at least 50 % by weight of the composition. Bringing the pH into the range of 1.5-4.5 hampers the development of onychomycosis. The acid can be incorporated in for instance a cream of lotion. The physiologically acceptable carrier should be selected for not neutralising the effect of the acid.

The physiologically acceptable acid is a carboxylic acid selected from the group consisting of lactic acid, malic acid, tartaric acid, citric acid, acetic acid, propionic acid, isopropionic acid, oxalic acid, glutaric acid, adipic acid and glycolic acid. Carboxylic acids are capable of achieving the desired pH drop on the infected skin and/or nails, and are typically well tolerated by the treated person.

The carboxylic acid is elected from the group consisting of lactic acid, malic acid, tartaric acid, citric acid, acetic acid, propionic acid, isopropionic acid, oxalic acid, glutaric acid, adipic acid and glycolic acid.

Most preferably, the carboxylic acid is lactic acid. Lactic acid gives the desired result against onychomycosis and is well tolerated by persons.

Another preferred carboxylic acid is acetic acid. Acetic acid gives good results against onchyomycosis, is well tolerated. As acetic acid is a relatively cheap compound, in particular compared to lactic acid, acetic acid is a good alternative for lactic acid, and may also be used in an acetic acid/lactic acid combination in order to diminish the amount of lactic acid used while retaining the desired skin or nail acidifying effect.

The carboxylic acid is present in a quantity of at least 1% by weight. In this quantity, the development onychomycosis is effectively hampered.

Composition according to any of the preceding claims, wherein the composition comprises at least 10% by weight of lactic acid. In this quantity, the development onychomycosis is severely hindered and in some cases even stopped.

The composition also comprises at least one C1-C4 alkyl ester of lactic acid. The molecular ratio free acid to the ester can be at least 1:100, preferably at least 1:20, most preferably at least 1:10. Such C1-C4 alkyl esters are excellent carriers for the acid.

In a preferred embiodiment, the composition comprises lactic acid as the physiologically acceptable acid, and lactic acid ethyl ester as a physiologically acceptable carrier.

In another preferred embodiment, the composition comprises acetic acid as the physiologically acceptable acid, and lactic acid ethyl ester as a physiologically acceptable carrier.

It is also possible to obtain a satisfactory composition by mixing lactic acid and acetic acid as physiologically acceptable acids, using lactic acid ethyl ester as a physiologically acceptable carrier.

The invention relates to the use of a composition according to the invention for the preparation of a product for the treatment of fungal infections.

The invention also relates to the use of a composition according to the invention for the preparation of a product for the treatment of fungal infections in keratinous tissue.

The invention further relates to the use of a composition according to the invention for the preparation of a product for the treatment of onychomycosis.

The invention moreover relates to the use of a composition for the preparation of a product for the treatment of fungal infections in keratinous tissue.

The invention also relates to the use of a composition capable of lowering the pH of the human skin and/or nails to the range of 1.5-4.5 for the preparation of a product for the treatment of fungal infections.

The invention relates to a device, comprising a container comprising a composition according to the invention, and an applicator connected to the container, wherein the applicator is adapted to apply the composition from the container to a part of human skin and/or nails to be treated.

The invention further relates to a kit of parts, comprising at least one container comprising a composition according to the invention and at least one scrub device for scrubbing a skin and/or nails part treated with the composition.

The invention provides a composition for topical application, including an effective amount of lactic acid. The combination of lactic acid, or a similar acid, or a derivative thereof and at least one physiologically acceptable carrier to aid penetration into the nail were shown to yield an improved effect in the treatment of fungal infections, in particular the treatment of onychomycosis. It is postulated that fungal infections benefit from the ability of lactic acid in controlling the pH of the nail, an essential part of this formula to combat nail fungus. This is enhanced by presence of the C1-C4 alkyl ester of lactic acid as a carrier and stabilizer, which improves the penetration of lactic acid or its derivatives into the skin or nail.

The lactic acid is preferably mixed homogeneously with the C1-C4 alkyl ester of lactic acid. Most preferably, the lactic acid is dissolved in the C1-C4 alkyl ester of lactic acid. The C1-C4 alkyl ester of lactic acid is present as a major component of the composition, in a quantity of at least 50% by weight of the composition, preferably at least 90%.

The composition may include additional suitable components, for instance fragrances, emulsifiers, detergents, antioxidants and preservatives, and other ingredients commonly used in pharmaceutical and cosmetic formulations. Preferably, the composition is essentially free of water, which increases the stability of the composition over time. Preferably, the composition is formulated as a fluid composition such as a cream, or more preferably as a liquid composition such as a tonic, which is relatively easy to apply to the human skin and/or nails.

The C1-C4 esters include methyl, ethyl, n-propyl, isopropyl, n-butyl, isobutyl, sec-butyl and tert-butyl esters. Ethyl esters are preferred. The C1-C4 alkyl ester is derived from lactic acid.

The C1-C4 alkyl ester is derived from lactic acid. The C1-C4 esters of this acid is particularly effective. The ethyl esters of this compound is preferred. C1-C4 alkyl esters of lactic acid was shown to be the most versatile.

In a preferred embodiment, the C1-C4 alkyl ester is lactic acid ethyl ester. This compound showed the best results.

In a preferred embodiment, the composition comprises at least 1% by weight of lactic acid. Such amounts have an advantageous effect on fungal infections, in particular onychomycosis. The amount may be optimized for a specific fungal infection.

It is preferred if the composition also comprises the free acid derived from the lactic acid used in the C1-C4 alkyl ester in a molecular ratio of at least 1:100 with respect to the C1-C4 alkyl ester, preferably at least 1:20, most preferably at least 1:10. Thus, a more stable composition is achieved. The availability of the carboxylic acid prevents the reversal of the esterification reaction in the presence of water, resulting in the free carboxylic acid and alcohol. For instance, a composition according to the invention, based on lactic acid ethyl ester, could be stabilized by adding lactic acid in a ratio of at least 1:100, preferably at least 1:20, most preferably at least 1:10

In a preferred embodiment, the composition comprises at least 50% by weight C1-C4 lactic acid ethyl ester, lactic acid in a ratio of at least 1:100 with respect to lactic acid ethyl ester, and at least 1% by weight of lactic acid.

Preferably, the composition also comprises a nail-penetrating agent. A nail penetrating agent allows the acid and optional other active ingredients to permeate into the nail, which was found to lead to a better inhibition of the growth of nail infections, in particular fungal nail infections.

Preferred nail-penetrating agents are selected from the group consisting of urea, dimethyl isosorbide and ethyl lactate, C1-C4 alkyl ester of lactic acid, preferably ethyl lactate. Mixtures of various nail penetrating agents may be used advantageously.

The invention relates to the use of a composition according to the invention for the preparation of a product, for instance a medicament, for the treatment of fungal infections. Fungal infections the composition according to the invention may be used against include onychomycosis and other microbiological infections. Typically, the amount of lactic acid or derivatives thereof are within 1-30% by weight.

The invention also relates to a device, comprising a container comprising a composition according to the invention, and an applicator connected to the container, wherein the applicator is adapted to apply the composition from the container to a part of human skin to be treated. Thus, the composition according to the invention is easy to apply and store.

The invention further relates to a kit of parts, comprising at least one container comprising a composition according to the invention and at least one scrub device for scrubbing a skin part treated with the composition, optionally also comprising a skin cream. Thus, skin conditions can be treated with a composition according to the invention and subsequently scrubbed mechanically by the scrub device.

### BRIEF DESCRIPTION OF THE DRAWINGS

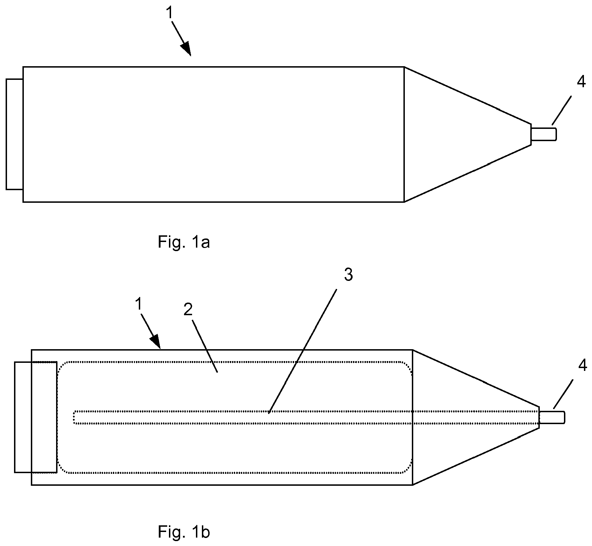

Figure 1 shows an applicator for applying a composition according to the invention.

### DESCRIPTION OF PREFERRED EMBODIMENTS

The invention will now be further elucidated by the following non-limiting examples. Applicator device
Figure 1 shows an application device, somewhat resembling a felt-tipped type marker. The device comprises a reservoir containing a composition according to the invention.
The device is pencil-shaped, and suitable to be hand-held. The reservoir is provided with an absorbing element made of a liquid-absorbing material capable of capillary action. This absorbing element dips into the liquid composition and extends from the inner part reservoir to the outside of the reservoir. The liquid contained in the reservoir within the marker and would be applied via a tip connecting to or made out of the distal end of the absorbing element extending out of the reservoir. Due to capillary action, the tip remains moist with the liquid product. For such an applicator device, the viscosity of the liquid composition will have to be sufficiently low. The device may be provided with a cap to prevent volatile solvents of the composition to evaporate and dry the tip out. The moist tip is contacted with the skin part to be treated, preferably while applying some pressure, in order to apply the composition from the reservoir.

The reservoir may be filled with different preferred embodiments according to the invention, depending on the envisaged skin condition to be treated. For all of these applications it was shown that the combination of lactic acid dissolved in or mixed with at least one physiologically acceptable C1-C4 alkyl ester of lactic acid as a carrier yielded a better absorbance of lactic acid into the skin than a comparable aqueous composition with the same lactic acid concentration.

All components are commercially available; the compositions were prepared using well-known mixing and blending techniques. pH measurements on the skin can be determined using commercially available pH meters, for instance the pH meter '1140' from Mettler Toledo.

### Example 1 : onychomycosis

An effective composition for treatment of onychomycosis was prepared by well know mixing techniques (percentages by weight).:

| | |
|---|---|
| lactic acid | 5% |
| lactic acid ethyl ester | 95% |

The resulting liquid is applied as a tonic, and showed a changed environment of the nail to the fungus disadvantage. The pH on the skin of treated persons temporarily dropped to a value in the range of 2-4. Such low pH environments appear to hamper the development of onychomycosis. Over time this effectively treated the nail of nail fungus.

### Example 2: onychomycosis

An effective composition for treatment of onychomycosis was prepared by well know mixing techniques (percentages by weight):

| | |
|---|---|
| Melaleuca Alternifolia | 1 % |
| Lavendula Officinalis | 2% |
| Callitris Intratropica | 2% |
| lactic acid | 2% |
| lactic acid ethyl ester | 93% |

The resulting liquid is applied as an tonic, and showed a changed the environment of the nail to the fungus disadvantage, showing a temporary pH drop of the skin to approximately 3-4. Over time this effectively treated the nail of nail fungus. This example is cosmetically more attractive than example 1.

### Example 3: Microbial infections of the skin.

An effective composition against warts was prepared by well know mixing techniques (percentages by weight):

| | |
|---|---|
| lactic acid | 20% |
| lactic acid ethyl ester | 80% |

Upon application to the skin, the pH of the skin dropped to approximately 2. Various types of microbial infections were successfully treated using this composition, resulting in complete or partial removal of the infection after multiple treatments.

### Example 4: onychomycosis

An effective composition for treatment of onychomycosis was prepared by well know mixing techniques (percentages by weight).:

| | |
|---|---|
| acetic acid | 5% |
| ethyl acid ethyl ester | 95% |

The resulting liquid is applied as a tonic, and showed a changed environment of the nail to the fungus disadvantage. The pH on the nail of treated persons temporarily dropped to a value in the range of 2-4. Such low pH environments appear to hamper the development of onychomycosis. Over time this effectively treated the nail of nail fungus. The same composition using a mixture of 1% lactic acid and 4% acetic acid instead of 5% acetic acid also yielded satisfactory results against onchyomycosis.

### Example 5: onychomycosis

An effective composition for treatment of onychomycosis was prepared by well know mixing techniques (percentages by weight):

| | |
|---|---|
| lactic acid | 15% |
| urea | 5% |
| lactic acid ethyl ester | 80% |

The resulting liquid is applied as an tonic, and showed a changed the environment of the nail to the fungus disadvantage, showing a temporary pH drop of the nail to approximately 3-4. Over time this effectively treated the nail of nail fungus. This example is cosmetically more attractive than example 1.

### Example 6: Microbial infections of the nail.

An effective composition against nail microbial infections was prepared by well know mixing techniques (percentages by weight):

| | |
|---|---|
| acetic acid | 15% |
| dimethyl isosorbide | 10% |
| acetic acid ethyl ester | 75% |

Upon application to the skin, the pH of the nail dropped to approximately 2.5. Dimethyl isosorbide acts as a nail-penetrating agent, enhancing the penetration by acetic acid. Various types of microbial infections were successfully treated using this composition, resulting in complete or partial removal of the infection after multiple treatments.

The compositions shown in the examples above may be used as such, or may be processed further to a final product, for instance by diluting with water or other solvents, or incorporation into a cream or lotion.

## Claims

1. Composition for the treatment of fungal infections, comprising:
• at least one physiologically acceptable carboxylic acid capable of reducing the pH in keratinous tissue, in particular nails, of a treated person below 4.5, preferably in the range of 1.5 - 4.5, wherein said at least one carboxylic acid is selected from the group consisting of lactic acid, malic acid, tartaric acid, citric acid, acetic acid, propionic acid, isopropionic acid, oxalic acid, glutaric acid, adipic acid, and glycolic acid; and
• a physiologically acceptable carrier comprising at least one C1-C4 alkyl ester of lactic acid;
and wherein the carboxylic acid is present in a quantity of at least 1 % by weight, and wherein the C1-C4 alkyl ester is present in a quantity of at least 50 % by weight of the composition.

2. Composition according to claim 1, wherein the carboxylic acid is lactic acid.

3. Composition according to claim 1, wherein the carboxylic acid is acetic acid.

4. Composition according to claim 1, wherein the composition comprises at least 10% by weight of lactic acid and/or acetic acid.

5. Composition according to any one of claims 1 to 4, wherein the physiologically acceptable carrier is lactic acid ethyl ester.

6. Composition according to any one of claims 1 to 5 further comprising a nail penetrating agent.

7. Composition according to any of the preceding claims, wherein the composition is essentially free of essential oils.

8. Use of a composition according to any of the preceding claims for the preparation of a product for the treatment of fungal infections in keratinous tissue by reducing the pH in keratinous tissue, in particular nails, of a treated person below 4.5.

9. Use according to claim 8 wherein the product is for treatment of nail infections, in particular superficial, cutaneous and/or subcutaneous mycosis.

10. Use according to claim 9 wherein the product is for the treatment of onychomycosis or tinea.

## Patentansprüche

1. Zusammensetzung zur Behandlung von Pilzinfektionen, umfassend:
• mindestens eine physiologisch unbedenkliche Carbonsäure, die geeignet ist, den pH in keratinösem Gewebe, insbesondere Nägeln, einer behandelten Person unter 4,5, bevorzugt in dem Bereich von 1,5 - 4,5, zu senken, wobei die mindestens eine Carbonsäure ausgewählt ist aus der Gruppe, bestehend aus Milchsäure, Apfelsäure, Weinsäure, Zitronensäure, Essigsäure, Propionsäure, Isopropionsäure, Oxalsäure, Glutarsäure, Adipinsäure und Glykolsäure; und
• einen physiologisch unbedenklichen Träger, umfassend mindesten einen C1-C4-Alkylester von Milchsäure;
und wobei die Carbonsäure in einer Menge von mindestens 1 Gew.-% vorhanden ist, und wobei der C1-C4-Alkylester in einer Menge von mindestens 50 Gew.-% der Zusammensetzung vorhanden ist.

2. Zusammensetzung nach Anspruch 1, wobei die Carbonsäure Milchsäure ist.

3. Zusammensetzung nach Anspruch 1, wobei die Carbonsäure Essigsäure ist.

4. Zusammensetzung nach Anspruch 1, wobei die Zusammensetzung mindestens 10 Gew.-% Milchsäure und/oder Essigsäure umfasst.

5. Zusammensetzung nach einem der Ansprüche 1 bis 4, wobei der physiologisch unbedenkliche Träger Milchsäureethylester ist.

6. Zusammensetzung nach einem der Ansprüche 1 bis 5, ferner umfassend ein nageldurchdringendes Mittel.

7. Zusammensetzung nach einem der vorhergehenden Ansprüche, wobei die Zusammensetzung im Wesentlichen frei von ätherischen Ölen ist.

8. Verwendung einer Zusammensetzung nach einem der vorhergehenden Ansprüche zur Herstellung eines Produkts für die Behandlung von Pilzinfektionen in keratinösem Gewebe durch Senkung des pHs in keratinösem Gewebe, insbesondere Nägeln, einer behandelten Person unter 4,5.

9. Verwendung nach Anspruch 8, wobei das Produkt für die Behandlung von Nagelinfektionen, insbesondere oberflächlicher kutaner und/oder subkutaner Mykose ist.

10. Verwendung nach Anspruch 9, wobei das Produkt für die Behandlung von Onychomykose oder Tinea ist.

## Revendications

1. Composition destinée au traitement des infections fongiques, comprenant :
• au moins un acide carboxylique physiologiquement acceptable capable d'abaisser le pH dans le tissu kératinique, en particulier dans les ongles de la personne traitée, en dessous de 4,5, de préférence dans la plage de 1,5 à 4,5,
ledit au moins un acide carboxylique étant choisi dans le groupe constitué de l'acide lactique, de l'acide malique, de l'acide tartrique, de l'acide citrique, de l'acide acétique, de l'acide propionique, de l'acide isopropionique, de l'acide oxalique, de l'acide glutarique, de l'acide adipique et de l'acide glycolique ; et
• un véhicule physiologiquement acceptable comprenant au moins un ester d'alkyle en C₁-C₄ de l'acide lactique ;
et dans laquelle l'acide carboxylique est présent dans une quantité d'au moins 1 % en poids et l'ester d'alkyle en C₁-C₄ est présent dans une quantité d'au moins 50 % en poids de la composition.

2. Composition selon la revendication 1, dans laquelle l'acide carboxylique est l'acide lactique.

3. Composition selon la revendication 1, dans laquelle l'acide carboxylique est l'acide acétique.

4. Composition selon la revendication 1, la composition comprenant au moins 10 % en poids d'acide lactique et/ou d'acide acétique.

5. Composition selon l'une quelconque des revendications 1 à 4, dans laquelle le véhicule pharmaceutiquement acceptable est l'ester éthylique de l'acide lactique.

6. Composition selon l'une quelconque des revendications 1 à 5 comprenant en outre un agent pénétrant dans les ongles.

7. Composition selon l'une quelconque des revendications précédentes, la composition étant essentiellement exempte d'huiles essentielles.

8. Utilisation d'une composition selon l'une quelconque des revendications précédentes pour la préparation d'un produit destiné au traitement des infections fongiques dans le tissu kératinique en abaissant le pH en dessous de 4,5 dans ce tissu kératinique, en particulier dans les ongles de la personne traitée.

9. Utilisation selon la revendication 8, le produit étant destiné au traitement des infections des ongles, en particulier des mycoses superficielles, cutanées et/ou sous-cutanées.

10. Utilisation selon la revendication 9, le produit étant destiné au traitement de l'onychomycose et du pied d'athlète.
